Europäisches Patentamt

European Patent Office

Office européen des brevets

(19)

(11) Numéro de publication: **0 295 265**
**B1**

(12) **FASCICULE DE BREVET EUROPEEN**

(45) Date de publication du fascicule du brevet: **23.01.91**

(51) Int. Cl.⁵: **A 61 M 5/28**

(21) Numéro de dépôt: **87902470.1**

(22) Date de dépôt: **23.03.87**

(86) Numéro de dépôt international:
**PCT/EP87/00163**

(87) Numéro de publication internationale:
**WO 87/06141 22.10.87 Gazette 87/23**

(54) **DISPOSITIF DE CONDITIONNEMENT DE SUBSTANCES LIQUIDES OU LIQUIDES ET SOLIDES.**

(30) Priorité: **10.04.86 CH 1413/86**

(43) Date de publication de la demande:
**21.12.88 Bulletin 88/51**

(45) Mention de la délivrance du brevet:
**23.01.91 Bulletin 91/04**

(84) Etats contractants désignés:
**AT BE CH DE FR GB IT LI LU NL SE**

(56) Documents cités:
**US-A-2 576 951**
**US-A-3 674 028**

(73) Titulaire: **MEDICORP HOLDING S.A.**
**11 Boulevard du Prince Henri**
**L-2014 Luxembourg (LU)**

(72) Inventeur: **MEYER, Gabriel**
**10A, chemin des Princes**
**CH-1222 Vésenaz (CH)**
Inventeur: **HOWALD, Ernst**
**10B, chemin des Princes**
**CH-1222 Vésenaz (CH)**

(74) Mandataire: **Nithardt, Roland**
**CABINET ROLAND NITHARDT Attn: Cabinet**
**MOSER & CIE Rue Edouard Verdan 15**
**CH-1400 Yverdon-les-Bains (CH)**

EP 0 295 265 B1

Courier Press, Leamington Spa, England.

**Description**

La présente invention concerne un dispositif de conditionnement de substances liquides ou liquides et solides, comprenant une ampoule de forme générale cylindrique allongée de section circulaire, à deux compartiments disposés dans le prolongement l'un de l'autre, séparés par un bouchon intermédiaire mobile en élastomère, et contenant chacun une substance différente, les deux substances devant être mises en présence l'une avec l'autre pour constituer un mélange liquide à usage médical ou paramédical, ce dispositif étant conçu premièrement pour assurer pendant un premier intervalle de temps, des conditions de stockage de chacune de ces substances, deuxièmement pour permettre, pendant un second intervalle de temps, leur mise en présence, et troisièmement pour permettre, au cours d'un troisième intervalle de temps, l'utilisation de ce mélange liquide évacuée hors du dispositif de conditionnement, dans lequel l'ampoule est fermée à une de ses extrémités, ouverte à son autre extrémité et comporte au moins une première zone cylindrique de section circulaire rétrécie pour définir un rétrécissement à l'intérieur de cette ampoule et au moins une deuxième zone cylindrique de section circulaire plus large que la section de la premièr zone; dans lequel, pendant le premier intervalle de temps correspondant à la phase de stockage, ladite zone cylindrique de section circulaire rétrécie est obturée d'une part de façon étanche par ledit bouchon intermédiaire mobile de manière à ménager à l'interieur de l'ampoule un premier compartiment disposé entre l'extrémité fermée de cette ampoule et ledit bouchon intermèdiaire mobile et un second compartiment disposé entre ledit bouchon intermédiaire mobile et l'extrémité ouverte de cette ampoule, et d'autre part par un piston-vanne agencé pour fermer l'extrémité ouverte de l'ampoule; dans lequel, pendant ledit second intervalle de temps correspondant à la phase de mise en presence des deux substances, ledit bouchon intermédiaire mobile est refoulé de la zone cylindrique de section circulaire retrécie dans une zone de section circulaire plus large pour permettre la communication entre le premier et le second compartiment et la mise en présence en milieu fermé des substances qu'ils contiennent, ladite zone cylindrique de section circulaire rétrécie restant obturée par le piston-vanne, et dans lequel, pendant ledit troisième intervalle de temps, ledit piston-vanne est poussé à l'intérieur de l'ampoule pour provoquer l'evacuation du mélange liquide hors de l'ampoule.

Le brevet US—A—2,576,951 décrit une seringue qui correspond au dispositif de conditionnement mentionné ci-dessus. L'ampoule est pourvue d'un étranglement dans lequel est engagé un bouchon intermédiaire mobile. L'extrémité ouverte de l'ampoule est équipée d'un obturateur qui doit être perforé par une aiguille à deux pointes, avant d'être enfoncé à l'intérieur de l'ampoule au moyen de l'injecteur prevu pour être adapté sur l'ampoule. Ce système présente de nombreux inconvénients. La perforation de l'obturateur par une aiguille engendre des particules qui risquent d'être injectées dans le corps du patient. Le volume mort qui correspond à la quantité de liquide résiduel dans la seringue aprè l'injection est relativement important. La mise en place précise et le maintien en position du bouchon intermédiaire mobile dans la zone de l'étranglement de l'ampoule sont délicats.

le brevet americain No. 3,563,415 décrit un distributeur de liquide du type goutte à goutte, comportant un flacon à deux compartiments dont l'un contient une poudre et l'autre un solvant liquide, les deux substances etant destinees à étre mélangées pour constituer un medicament liquide. Ces deux compartiments sont séparés par un bouchon intermédiaire bloqué en position dans un col étroit, pendant la phase ce stockage di distributeur. Ce col contient par ailleurs un obturateur composé d'un premier élément susceptible de coulisser à l'intérieur de ce col, pourvu d'un canal radial et d'un second élément agence pour s'adapter sur le premier élément pour obturer ce canal radial. Le compartiment contenant la poudre présente un diamètre sensiblement plus grand que celui du col. Au moment de l'utilisation, l'obturateur est enfonce dans le col. La pression exercee par l'intermédiaire du solvant liquide contenu dans l'un des compartiments refoule le bouchon intermédiaire dans le compartiment contenant la poudre. Ce bouchon intermédiaire tombe par gravité dans ce compartiment et le mélange peut s'effectuer. Lorsque la poudre est complètement dissoute par le liquide, le second élément de l'obturateur est enleve, ce qui débouche le canal axial et permet l'écoulement au médicament.

Ce dispositif ne se prête pas à une utilisation comme seringue d'injection ou comme pulvérisateur. Le seul usage prévu est une utilisation comme dispositif de conditionnement à deux compartiments, le médicament liquid résultant du mélange des deu composants s'écoulant par gravité hors du flacon.

La demande de brevet européen publiée sous le No. 0 144 483 décrit une seringue du type seringue mélangeuse, comportant un corps de seringue divise, pendant la phase de stockage, en deux compartiments par un bouchon intermédiaire. Le corps de cette seringue présente une forme sensiblement cylindrique et son extrémité distale porte une aiguille alors que son extrémité postérieure est obturée par la tête d'un piston. Le corps de la seringue comporte, sensiblement en son milieu, une déformation latérale, par exemple un renflement convexe, définissant une trajectoire de dérivation permettant le transfert d'un liquide contenu dans le compartiment postérieur vers le compartiment antérieur ou chambre de mélange, lorsque le bouchon intermédiaire est refoulé à la hauteur de cette deformation.

Lorsque le composant contenu pendant la phase de stockage dans le compartiment antérieure est un lyophilisat, la substance originale à lyophiliser, qui se présente habituellement sous forme liquide, est en fait contenue dans un compartiment délimité d'une part par les parois à l'intérieur du corps de la seringue et d'autre part par une surface du bouchon intermérdiaire. Pour permettre l'évacuation des vapeurs engendrees au course de l'opération de lyophilisation, l'extrémité distale de la seringue reste ouverte alors que son autre extrémité doit être obturée de façon étanche par le bouchon intermédiar. Cette étanchéité est difficile à réaliser pendant la phase de lyophilisation qui s'accompagne, de manière connue, d'une congélation au cours de laquelle le bouchon d'obturation en élastomère perd toures ou partie de ces propriétés d'élasticité. Un des inconvénients du système décrit provient de ce que la substance à lyophiliser est obligatoirement en contact avec le verre du corps de seringue et l'élastomère du bouchon intermédiaire pendant la phase de lyophilisation et du fait que l'étanchéité entre ces deux matériaux est difficile à préserver au cours d'une congélation qui accompagne obligatoirement l'opération de lyophilsation.

En outre, des moyens doivent être prévus pour obturer l'extrémité distale de la seringue équipée d'une aiguille qui débouche directement dans le compartiment antérieur contenant initialement le lyophilisat et servant de chambre de mélange.

La présente invention se propose de pallier les inconvénients des systemes connus decrits ci-dessus en proposant un dispositif de conditionnement à deux compartiments permettant d'effectuer en milieu ferme le mélange de deux substances respectivement contenues dans les deux compartiments, et d'utiliser ce mélange comme substance injectable au moyen d'une seringue, substance à pulvériser au moyen d'un pulvérisateur ou substance à déverser goutte à goutte ou par jet.

Un autre avantage du dispositif est que toutes les opérations de remplissage et de mise en place des différents éléments peuvent être effectuees automatiquement en très grande série, les ampoules cheminant côte-à-côte sans aucun support intermédiaire sur des bandes ou des plateaux transporteurs destinés à les amener dans differents postes de remplissage et/ou d'assemblage.

En outre, toutes les fonctions requises au cours du stockage et pendant la phase d'utilisation, notamment l'étanchéité respective des deux compartiments pendant le stockage et l'effet piston destiné à ejecter le mélange hors de la chambre de mélange pendant la phase d'utilisation sont réalisées par deux éléments, à savoir un bouchon intermédiaire mobile et un piston-vanne qui coopère avec une ampoule fermée à une extrémité aux formes particulières. Il en résulte une grande simplicité de fabrication et une grande sécurité d'utilisation.

Ce but est atteint par le dispositif de conditionnement selon l'invention caractérisé en ce que chaque section transversale du bouchon intermédiaire mobile présente, à l'état détendu dans la zone cylindrique de section circulaire large, une forme differente de la forme circulaire, une surface inférieure à la surface de la section transversale de ladite zone de section circulaire large, et au moins une dimension transversale égale au diametre de ladite zone de section circulaire large, et en ce que le bouchon intermédiaire mobile présente, à l'état comprimé dans la zone cylindrique de section circulaire rétrecie, une forme circulaire dont le diamètre est égal à celui de la section transversale de cette zone.

Selon une première forme de réalisation, l'ampoule comporte une seuile zone cylindrique de section circulaire rétrécie ménagée du côté de son extrémité ouverte et une seule zone cylindrique de section circulaire large ménagée du côté de son extrémité fermée cette zone cylindrique de section circulaire rétrécie comprenant essentiellement ledit second compartiment et cette zone cylindrique de section circulaire large comprenant essentiellement ledit premier compartiment.

Selon une variant de cette première réalisation, l'ampoule comporte une zone cylindrique unique de section circulaire rétrécie ménagée entre une premier zone cylindrique de section circulaire large et une seconde zone cylindrique de section circulaire large, ladite zone cylindrique de section circulaire rétrécie comprenant essentiellement ledit second compartiment et la seconde zone cylindrique de section circulaire large comprenant essentiellement ledit premier compartiment.

Dans cette variante, ladite zone cylindrique de section circulaire rétrécie peut comporter trois secteurs dont le premier à un diamètre adapte pour assurer un blocage étanche du piston-vanne pendant la phase de stockage, dont le deuxième définit ledit second compartiment et dont le dernier a un diamètre adapté pour assurer un blocage étanche du bouchon intermédiaire mobile pendant cette même phase de stockage.

Selon une autre forme de réalisation l'ampoule comporte une première zone cylindrique de section circulaire large, une première zone cylindrique de section circulaire rétrécie, une seconde zone cylindrique de section circulaire large, une seconde zone cylindrique de section circulaire rétrécie et une troisième zone cylindrique de section circulaire large, la première zone cylindrique de section circulaire rétrécie ayant un diamètre adapté pour assurer un blockage étanche du piston-vanne pendant la phase de stockage et la seconde zone cylindrique de section circulaire rétrécie ayant un diamètre adapte pour assurer un blocage étanche du bouchon intermédiaire mobile pendant cette même phase, les zones cylindriques de section circulaire large definissant respectivement ledit second et ledit premier compartiment.

Le bouchon intermédiaire mobile présente avantageusement une forme générale cylindrique et sa surface latérale a une section au moins approximativement polygonale à l'état détendu.

Selon un mode de réalisation préféré, le bouchon intermédiaire mobile présente une forme générale cylindrique et sa surface latérale a une section approximativement ovale à l'état détendu.

Ces constructions peuvent également être appliquées à l'organe d'obturation qui peut présenter une forme générale cylindrique et sa surface latérale peut avoir une forme au moins approximativement polygonale à l'état détendu ou une forme au moins approximativement ovale dans ce même état.

Pour faciliter l'évacuation de vapeurs pendant l'opération de lyophilisation, le bouchon intermédiaire mobile peut présenter une entaille ménagée sur une partie de sa hauteur, cette entaille débouchant dans la surface disposée en regard du fond de l'ampoule.

La présente invention sera mieux comprise en référence à la description d'un exemple de réalisation et du dessin annexe dans lequel:

La figure 1 représente une vue en coupe axiale du dispositif de conditionnement selon l'invention dans sa position de stockage,

La figure 2 représente une première phase de remplissage de l'ampoule,

La figure 3A est une vue en coupe axiale illustrant une seconde phase de fabrication du dispositif, notamment lorsque l'une des substances conditionnées est lyophilisée,

Les figures 3B et 3C sont des vues en plan, de dessus de l'ampoule telle que représente par la fig. 3A,

La figure 4 représente une phase ultérieure de remplissage de l'ampoule, à la fin de la lyophilisation d'une des substances qu'elle contient,

La figure 5 représente la phase de mise en place du bouchon intermédiaire mobile après remplissage du premier compartiment l'ampoule,

La figure 6 illustre la phase de remplissage du second compartiment de l'ampoule,

La figure 7 illustre la mise en place de l'injecteur,

La figure 8 est une vue en coupe longitudinale partielle illustrant la phase d'utilisation ou dispositif de conditionnement après mélange des deux substances respectivement contenues dans les deux compartiments de l'ampoule,

La figure 9 illustre le dispositif de conditionnement utilisé comme seringue d'injection,

La figure 10 illustre le dispositif de conditionnement utilisé comme pulvérisateur, et

La figure 11 représenté une vue en coupe axiale d'une autre forme de réalisation de l'ampoule du dispositif de conditionnement selon l'invention.

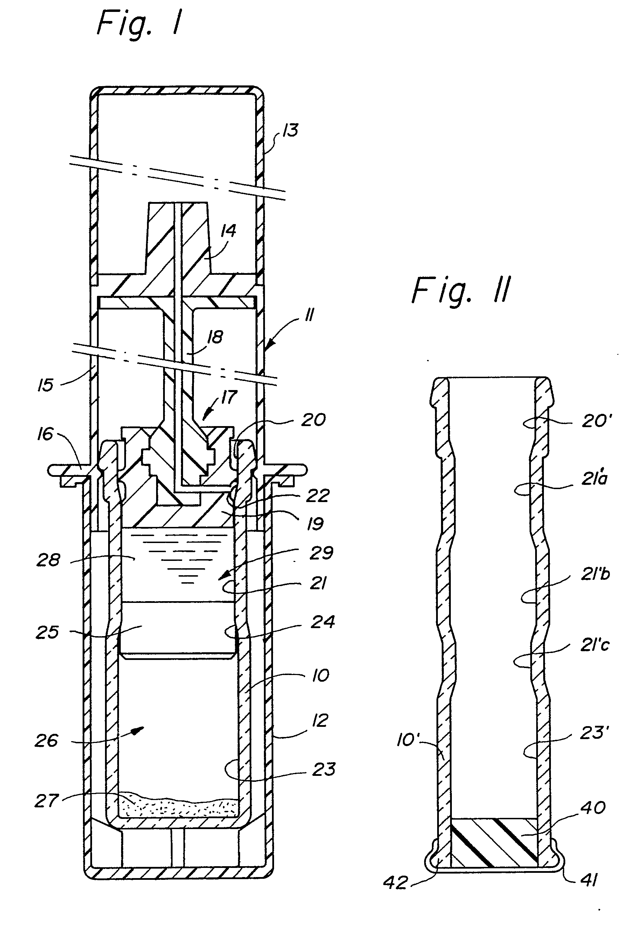

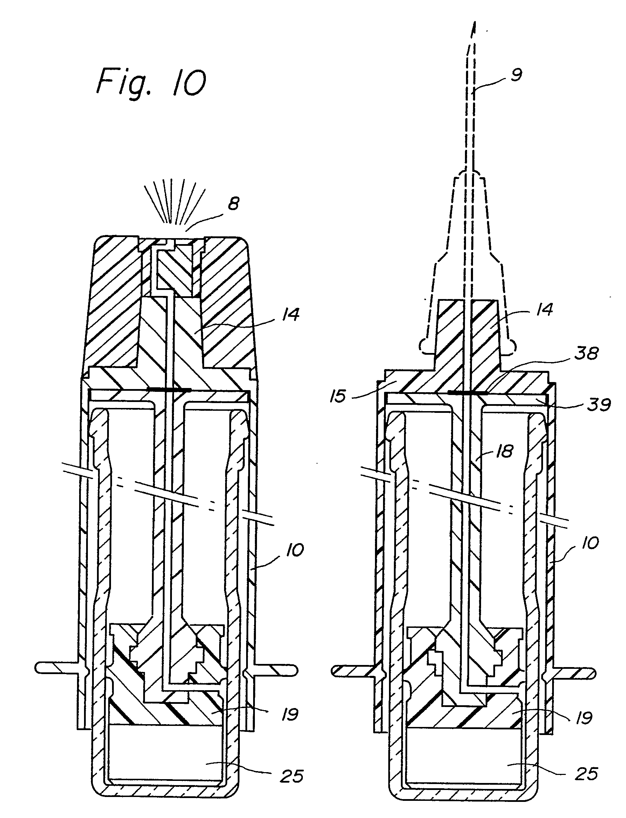

En référence aux figures 1 à 10 le dispositif de conditionnement à deux compartiments de substances à usage médical ou paramédical, tel que decrit plus en détail ci-dessous, comporte essentiellement une ampoule 10 de forme générale cylindrique, un injecteur 11 associé à cette ampoule, un cache-ampoule 12 couple à l'injecteur 11 et recouvrant sensiblement toute l'ampoule et un cache-embout 13, également couplé à l'injecteur 11, destiné à protéger un embout 14 solidaire d'une extrémité de l'injecteur 11. Selon les variantes réalisées, l'embout d'injection peut porter une aiguille 9 comme le montre plus particulièrement la fig. 9 et le dispositif de conditionnement devient alors une seringue mélangeuse préremplie ou une buse de pulvérisation 8 comme le montre plus particulièrement la fig. 10, et le dispositif de conditionnement devient alors un pulvérisateur du type nasal ou un dispositif compte-gouttes ou un bec verseur, etc...

L'injecteur 11 comporte une capsule 15 comprenant un manchon cylindrique fermé à une extrémité par un fond solidaire de l'embout porte-aiguille 14 et ouvert à son extrémité opposée dans laquelle est emboîtée l'extrémité ouverte de l'ampoule 10. Cette extrémité ouverte de la capsule 15 porte deux ailettes 16 utilisées de façon connue en soi au moment de l'injection. L'injecteur comporte par ailleurs un piston-vanne 17 monté à l'intérieur de la capsule 15 et fixé à son fond, par exemple par soudure par micro-ondes. Ce piston-vannes se compose d'une tige de piston 18 portant un organe d'obturation 19 qui joue le rôle de tête de piston dans certaines phases d'utilisation qui seront décrites plus en détail ci-dessous.

L'ampoule 10 fermée à une de ses extrémités, comprend du côté de son extrémité ouverte une première zone cylindrique de section circulaire large 20 suivie d'une zone cylindrique 21 de section circulaire rétrécie reliée à la zone précédente 20 par une première zone de raccordement tronconique 22. La zone cylindrique de section circulaire rétrécie 21 est suivie d'une seconde zone cylindrique de section circulaire large 23 reliée à la précédente par une seconde zone de raccordement tronconique 24.

Dans la position de stockage illustrée par la fig. 1, un bouchon intermédiaire mobile 25 est partiellement engagée dans la zone cylindrique de section circulaire rétrécie, du côté de la seconde zone de raccordement tronconique 24. L'organe d'obturation 19 du piston-vanne 17 est partiellement engagé dans cette zone cylindrique de section circulaire rétrécie 21 du côté de la première zone de raccordement tronconique 22. De cette manière, le bouchon intermédiaire mobile 25 délimite avec la fond de l'ampoule un premier compartiment 26 appelé par la suite chambre de mélange, contenant au stockage un lyophilisat 27 ou toute autre substance destinée à être mélangée à une autre substance 28, par exemple un solvant liquide qui est contenu au stockage dans un second compartiment 29 disposé entre le bouchon intermédiaire mobile 25 et l'organe d'obturation 19. Pendant toute la phase de stockage, les deux compartiments sont séparés de façon étanche par le bouchon intermédiaire mobile 25 et les substances qu'ils contiennent sont isolées l'une de l'autre.

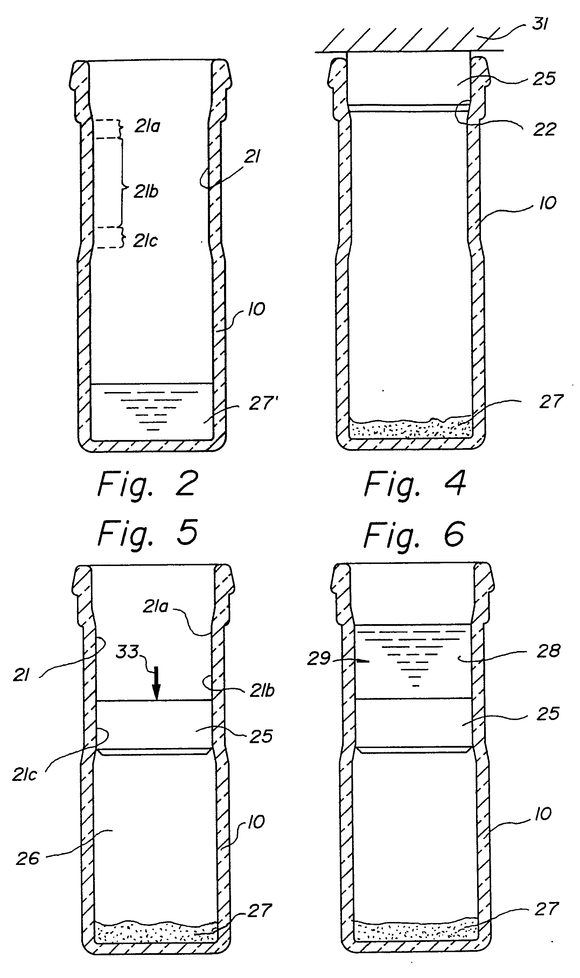

Les figures 2 à 8 permettront de comprendre les différentes phases de remplissage et d'assemblage qui aboutissent au dispositif de conditionnement à deux compartiments tel que représenté dans sa position de stockage par la fig. 1.

Dans l'exemple illustré, l'ampoule 10 est tout d'abord remplie d'une substance liquide 27' qui, après une opération de lyophilisation connue en soi, se transformera en un lyophilisat 27 contenu dans la chambre de mélange 26 de l'ampoule. La zone cylindrique de section circulaire rétrécie 21 comporte avantageusement trois secteurs 21a, 21b et 21c. Le secteur 21a est appelé le secteur de stockage étanche de l'organe d'obturation 19. Son diamètre est défini de telle manière qu'il assure un blocage étanche dudit organe d'obturation 19 pendant la phase de stockage du dispositif de conditionnement. Le secteur 21c est appelé secteur de stockage étanche du bouchon intermédiaire mobile 25. Son diamètre est défini de telle manière qu'il assure un blocage étanche dudit bouchon pendant toute la phase de stockage, ce blocage étant suffisamment étanche pour isoler parfaitement les substances respectivement contenues dans les deux compartiments. Le secteur central 21b est un secteur intermédiaire entre les deux secteurs de stockage. Son diamètre peut être égal à celui des deux secteurs de stockage 21a et 21c, mais cette condition n'est pas indispensable. La seule condition nécessaire est que le piston-vanne reste étanche pendant toute sa phase de déplacement à travers la zone cylindrique de section circulaire rétrécie, c'est-à-dire à travers les trois secteurs 21a, 21b et 21c. Le diamètre du secteur intermédiaire 21b peut être quelque peu plus grand que celui des secteurs 21a et 21c, de façon que le serrage exercé sur l'organe d'obturation 19 soit réduit dans ce secteur, mais ce diamètre ne peut en aucun cas dépasser une limite à partir de laquelle le piston-vanne perd son étanchéité.

Comme mentionné précédemment, le bouchon intermédiaire mobile 25 se trouve en partie engagé dans la seconde zone de raccordement tronconique 24 pendant la phase de stockage. Dans cette zone, la partie correspondante de ce bouchon est moins comprimée que la partie qui se trouve engagée dans la zone cylindrique de section circulaire rétrécie 21, et sera en mesure de reprendre très rapidement sa forme initiale dès que le bouchon intermédiaire mobile 25 sera poussé dans la zone cylindrique de section circulaire large. On verra par la suite, lors de la description du fonctionnement du système, qu'il est indispensable qu'au moins la partie du bouchon intermédiaire mobile orientée vers la chambre de mélange, exerce un frottement suffisant contre les parois de l'ampoule pour retenir le bouchon intermédiaire mobile en position dans cette chambre pendant la phase de transfer du solvant liquide 28 du second compartiment 29 dans la chambre de mélange. Cette retenue par friction est facilitée lorsqu'un phénomène de rémanence dû à un long stockage du bouchon intermédiaire mobile, ne ralentit pas le retour du bouchon, ou du moins d'une partie de ce bouchon à sont état partiellement détendu.

Le même probème se pose pour le piston-vanne et se résout de la manière suivante: seul l'organe d'obturation 19 est engagé dans une zone de forte compression, la languette périphérique disposée sous le canal radial (décrit plus en détail par la suite) étant localisée dans une zone de moindre compression, à savoir la première zone cylindrique de section circulaire large 20 ou la première zone de raccordement tronconique 22.

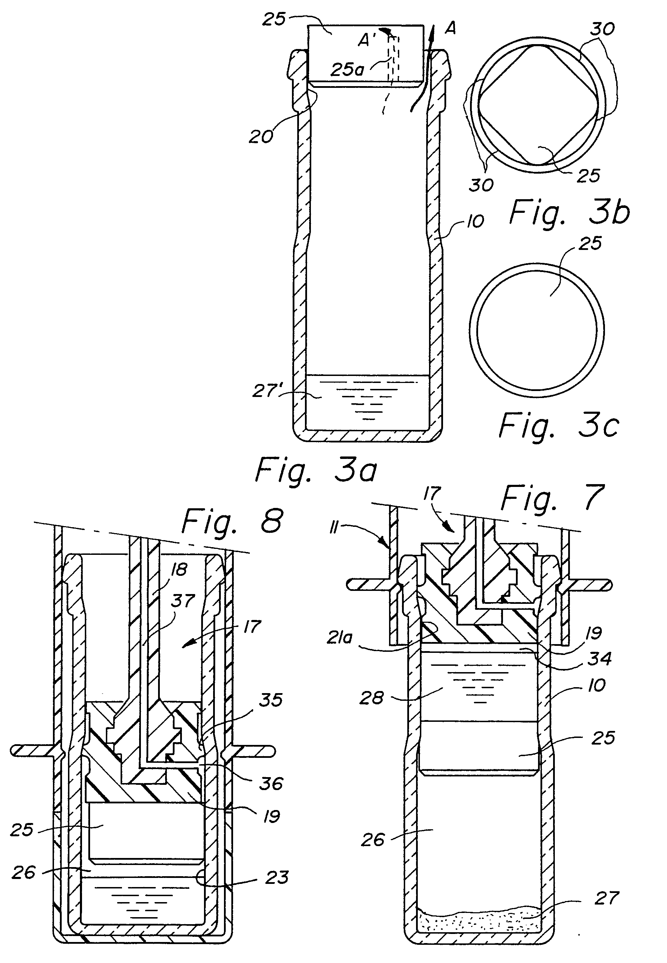

La figure 3A illustre le prépositionnement du bouchon intermédiaire mobile 25 en vue de la phase de lyophilisation. La fig. 3B montre une vue en plan du bouchon intermédiaire mobile 25 en place dans la première zone cylindrique de section circulaire large 20. La figure 3C représente le bouchon en place dans la zone cylindrique de section circulaire rétrécie 21 de l'ampoule 10. Ce bouchon est réalisé en une matière élastomère et est par conséquent déformable lorsqu'il est soumis à une contrainte. A l'état détendu, sa section est polygonale, par exemple carrée, de telle manière que des jours 30 apparaissent entre la paroi latérale du bouchon et la paroi intérieure de l'ampoule, lorsque ledit bouchon est engagé dans la première zone cylindrique de section circulaire large 20 de l'ampoule 10. La forme du bouchon intermédiaire mobile n'est cependant pas limitée à une forme polygonale. Il pourrait avoir initialement, c'est-à-dire à l'état détendu, une forme ovale, par exemple elliptique ayant un grand diamètre au moins légèrement supérieure au diamètre de la première zone cylindrique de section circulaire large 20 et un petit diamètre sensiblement inférieur à ce diamètre. A l'état comprimé, c'est-à-dire lorsqu'il est engagé dans la zone cylindrique de section circulaire rétrécie 21, le bouchon se déforme selon une forme circulaire dont le diamètre est égal à celui de cette section comme le montre plus précisément la figure 3C. Le serrage est suffisant pour garantir l'étanchéité.

Ces jours sont exploités pour permettre l'évacuation des vapeurs dans le sens de la flèche A au cours de l'opération de lyophilisation à laquelle est soumise la substance liquide 27'. A cet effet, le bouchon intermédiaire mobile peut comporter une entaille ou un évidement 25a destiné à faciliter l'évacuation des vapeurs, selon la flèche A', au cours de la lyophilisation.

Comme le montre la fig. 4, à la fin de cette opération, lorsque la substance originale 27' a été transformée en un lyophilisat 27, le bouchon intermédiaire mobile 25 est enfoncé dans l'ampoule 10 à une profondeur suffisante pour que son extrémité antérieure pénètre dans la première zone de raccordement tronconique 22 et obture cette ampoule de manière étanche. Ce premier enfoncement du bouchon intermédiaire mobile s'obtient grâce à une poussée ascendante exercée sur le corps de l'ampoule, le bouchon intermédiaire mobile 25 étant en appui contre la paroi supérieure 31 de l'enceinte de lyophilisation ou grâce à une poussée descendante exercée par cette paroi supérieure 31, ou une plaque mobile disposée à l'intérieur de l'enceinte de lyophilisation. Des moyens peuvent être prévus pour créer un vide partiel à l'intérieur de l'enceinte de lyophilisation à la fin de cette opération de sorte que ce vide règne également à l'intérieur de l'ampoule 10. A partir du moment où le bouchon intermédiaire mobile 25 est enfoncé dans le col de l'ampoule et assure une obturation étanche de cette dernière, le vide partiel subsistera à l'intérieur de cette ampoule, c'est-à-dire au-dessus du lyophilisat 27. Cette condition n'est cependant pas requise et une atmosphère contrôlée peut surmonter le lyophilisat, cette atmosphère étant par exemple constituée de gaz neutre.

Au cours d'une phase complémentaire illustrée par la fig. 5, le bouchon intermédiaire 25 est amené, éventuellement par des moyens mécaniques schématiquement représentés par la flèche 33, au voisinage de l'extrémité inférieure de la zone cylindrique de section circulaire rétrécie 21. Les moyens mécaniques 33 sont nécessaires dans certains cas, mais superflus dans d'autres. En effet, lorsque la pression atmosphérique est rétablie après la phase de lyophilisation, si un vide partiel approprie règne dans le compartiment 26, c'est-à-dire au-dessus du lyophilisat 27, la différence de pression peut éventuellement amener le bouchon intermédiaire 25 à coulisser sans assistance extérieure à travers le secteur intermédiaire 21b pour se bloquer en position dans le secteur de stockage étanche 21c.

La fig. 6 illustre la phase suivante qui consiste à introduire dans le compartiment supérieur 29 un solvant liquide 28 destiné à être mélangé au lyophilisat 27 pour reconstituer le médicament à injecter ou à faire absorber, par exemple par pulvérisation, à un patient.

La phase suivante illustrée par la fig. 7 consiste à monter l'injecteur 11 sur l'ampoule 10 en engageant l'organe d'obturation 19 du piston-vanne 17 dans le col de l'ampoule jusque dans le secteur 21a de la zone cylindrique de section circulaire rétrécie 21. Selon un mode de réalisation préféré, le remplissage de la chambre supérieure 29 au moyen du solvant 28 s'effectue dans une atmosphère d'anhydride carbonique dont un certain volume se trouve initialement emprisonné entre l'organe d'obturation 19 et la surface supérieure dudit liquide 28. Ce volume 34 est rapidement dissout dans le solvant liquide 28, ce qui a pour conséquence de créer une dépression qui provoque une remontée équivalente en volume du bouchon intermédiaire mobile 25. Cet état final est représenté par la fig. 1 et correspond à la position de stockage du dispositif de conditionnement.

Au moment de l'utilisation, on retire le cache-ampoule 12 que l'on remplace par le cache-embout 13. Ce dernier est dimensionné de telle manière qu'il suffit d'appuyer sur son fond plat en maintenant la seringue par les ailettes 16 pour amener l'ampoule progressivement dans la position représentée par la fig. 8. Ce mouvement provoque le déplacement du piston-vanne 17 vers l'intérieur de la zone cylindrique de section circulaire rétrécie 21. Le bouchon intermédiaire mobile sort de cette zone sous la pression intégralement transmise par le liquide 28 et pénètre dans la zone d'entrée de la chambre de mélange où il s'expanse en reprenant sa forme initiale représentée par la fig. 3B, c'est-à-dire la forme dans laquelle des jours 30 apparaissent entre sa surface latérale et les parois intérieures de l'ampoule. Les forces de frottement du bouchon intermédiaire mobile contre l'ampoule tendent à maintenir ce bouchon dans cette position, alors que les forces engendrées par la pression exercée par l'intermédiaire du liquide auraient tendance à le faire avancer dans la chambre de mélange. Les dimensions du bouchon intermédiaire mobile et celles des ouvertures, sont de préférence déterminées de telle manière que les forces de frottement soient supérieures aux forces de poussée, de sorte que le bouchon intermédiaire mobile 25 reste en place et que le liquide soit transféré du second compartiment dans la chambre de mélange à travers les jours 30. Le bouchon intermédiaire mobile 25 reste ainsi en position jusqu'à ce que tout le liquide soit transféré et jusqu'à ce que l'organe d'obturation 19 soit en appui contre sa surface supérieure. Le solvant liquide 28 dissout le lyophilisat contenu dans la chambre de mélange 26. La solution obtenue constitue le médicament à injecter, à pulvériser ou à déverser goutte à goutte ou sous forme de jet.

Après cette première phase au cours de laquelle le bouchon intermédiaire mobile est bloqué et le piston-vanne traverse la zone cylindrique de section circulaire rétrécie pour refouler le liquide du second compartiment à travers les jours 30, l'ensemble bouchon intermédiaire mobile et piston-vanne s'enfonce progressivement dans la chambre de mélange.

D'une façon connue par les brevets antérieurs du même déposant, le piston-vanne 17 comporte au moins une languette périphérique 35 dont le diamètre, à l'état détendu, est légèrement supérieur au diamètre de la seconde zone cylindrique de section circulaire large 23 pour garantir que le médicament soit refoulé à travers un conduit radial 36 ménagé dans l'organe d'obturation 19 et relié à un conduit axial 37 traversant la tige de piston 18 pour se raccorder à une aiguille 9 (voir fig. 9), un pulvérisateur 8 (voir fig. 10) ou tout autre dispositif approprié pour libérer le médicament. Lorsque l'opérateur enfonce l'ampoule en direction des ailettes de la capsule, le mélange est refoulé vers le canal radial à travers les jours 30.

En fin de course, le bouchon intermédiaire 25 est refoulé au fond de l'ampoule 10 et l'organe d'obturation 19 est appuyé contre la surface dudit bouchon intermédiaire mobile. Il est évident que la forme et les dimensions du bouchon intermédiaire mobile doivent être telle qu'il coulisse axialement sans basculer à l'interior de l'ampoule.

D'une manière également connue par les brevets antérieurs du même déposant, un filtre 38 peut être interposé entre une embase 39 portant la tige de piston 18 et le fond de la capsule 15.

Pour des raisons techniques, il peut être difficile de réaliser un col de section circulaire rétrécie relativement long. Dans ce cas, la zone cylindrique de section circulaire rétrécie 21 de l'exemple précédent, qui comporte trois secteurs 21a, 21b et 21c peu différenciés, peut être remplacée par trois zones nettement délimitées. Cette forme de réalisation est illustrée par la fig 11. L'ampoule 10', fermée à l'une de ses extrémités, comporte, du côté de son extrémité ouverte, une première zone cylindrique de section circulaire large 20' suivie successivement d'une première zone cylindrique de section circulaire rétrécie 21'a, d'une seconde zone cylindrique de section circulaire large 21'b, d'une seconde zone cylindrique de section circulaire rétrécie 21'c et d'une troisième zone cylindrique de section circulaire large 23'.

Au point de vue fonctionnement, la première zone cylindrique de section circulaire rétrécie 21'a correspond au secteur 21a de la zone cylindrique de section circulaire rétrécie 21 de l'ampoule 10, la seconde zone cylindrique de section circulaire rétrécie 21'c correspond au secteur 21c de la zone cylindrique de section circulaire rétrécie 21 de l'ampoule 10 et la seconde zone cylindrique de section circulaire large 21'b correspond au secteur intermédiaire 1b de la zone cylindrique de section circulaire rétrécie 21 de l'ampoule 10.

Dans le cas, le bouchon intermédiaire est prepositionné dans la seconde zone cylindrique 21'b de section circulaire large pendant la phase de lyophilisation, ce qui permet l'évacuation des vapeurs à travers les jours dus à la déformation élastique de ce bouchon, comme cela a été decrit précédemment.

Au cours de la période de stockage, l'organe d'obturation du piston-vanne est positionné dans la première zone cylindrique de section circulaire rétrécie 21'a et le bouchon intermédiaire est en place dans la seconde zone cylindrique de section circulaire rétrécie 21'c. Les diamètres des zones cylindriques de section circulaire large 21'b et 23' sont tels que le piston-vanne, et plus particulièrement la ou les languettes annulaires disposées sous le canal radial, remplissant leur rôle de "segments de piston" ou de "racleurs", en appui élastique contre les parois intérieures de l'ampoule, et empéchent toute fuite de liquide a injecter.

Comme precédemment, le bouchon intermédiaire est réalisé en élastomère et presente une forme polygonale, elliptique, régulière ou irrégulière, symétrique ou assymétrique qui présente des jours ou des ouvertures lorsqu'il est en place dans une zone cylindrique de section circulaire large et assure une étanchéité parfaite lorsqu'il est en place dans une zone cylindrique de section circulaire rétrécie. Dans la pratique, il pourrait également comporter une fente ou une ouverture centrale tendant à s'ouvrir et à se fermer selon que ce bouchon se trouve dans l'une ou l'autre des zones cylindriques mentionnées.

Les caractéristiques constructives du bouchon intermédiaire mobile pourraient également être appliquées à l'organe d'obturation du piston-vanne. En effet, une forme polygonale ou elliptique ou autre, à surface variable selon que l'organe se trouve dans une zone cylindrique de section circulaire rétrécie ou une zone cylindrique de section circulaire large, permettrait de réduire de façon conséquente le volume mort du système et de diminuer les problèmes de tolérances de diamètre de la chambre de mélange. Bien que on obligatoire, cette forme de réalisation garantit la sécurité du fonctionnement du piston-vanne dans toutes les conditions de tolérances de fabrication.

Lorsque l'une des substances est un lyophilisat, l'ampoule comporte avantageusement un fond fermé réalisé d'une pièce avec le corps, de preference en verre. Lorsque l'une des substances est une poudre très fine, l'ampoule comporte de préférence un fond obturé par un bouchon 40 (voir fig. 11) qui est fixé au moyen d'un sertissage 41 adapté sur un bourrelet annulaire 42 solidaire de l'extrémité correspondante de l'ampoule. Dans ce cas, la mise en place d'une poudre très fine dans le premier compartiment entraînerait nécessairement un dépôt de poudre le long des parois de l'ampoule, dépôt qui nuirait à l'étanchéite du bouchon intermédiaire mobile. En outre, la mise en place de ce bouchon intermédiaire mobile, lorsque la poudre est déjà introduite dans l'ampoule, provoquerait des turbulences soulevant une partie de la poudre déposée au fond et risquant également de nuire à l'étanchéité dudit bouchon. C'est la raison pour laquelle il est avantageux, dans ce cas, de mettre en place tout d'abord le bouchon intermédiaire mobile, puis le bouchon d'obturation 40 qui est ensuite fixé définitivement par sertissage, étant donné qu'il ne joue plus aucun rôle au cours du fonctionnement du système.

Dans toutes les variantes précédentes, la phase d'utilisation comprend deux etapes successives: au cours d'une première étape, le composant liquide passe du second compartiment dans la chambre de mélange qui est en fait le compartiment le plus éloigné de l'extrémité ouverte de l'ampoule, et au cours d'une seconde étape le mélange liquide passe du premier compartiment à travers les jours du bouchon intermédiaire mobile pour être évacué à travers l'orifice unique de l'ampoule. On observe en conséquence un double transfert à travers le bouchon intermédiaire mobile, ce double transfert étant autorisé par la geométrie particulière du bouchon intermédiaire combiné à une géométrie particulière de l'ampoule.

Les avantages qui en résultent sont les suivants:

1. Le système ne comporte que deux éléments d'obturation actifs.

2. L'ampoule ne comporte qu'un orifice unique au cours de l'utilisation.

3. Aucun retournement n'est requis pendant toutes les phases de conditionnement.

4. Le mélange des deux composants s'effectue en milieu complètement fermé.

**Revendications**

1. Dispositif de conditionnement de substances liquides ou liquides et solides, comprenant une ampoule (10) de forme générale cylindrique allongee de section circulaire, à deux compartiments (26, 28) disposés dans le prolongement l'un de

EP 0 295 265 B1

l'autre, séparés par un bouchon intermédiaire mobile (25) en élastomère, et contenant chacun au moins une substance différente, les deux substances devant être mises en présence l'une avec l'autre pour constituer un mélange liquide à usage médical ou paramédical, ce dispositif étant conçu premièrement pour assurer, pendant un premier intervalle de temps, des conditions de stockage de chacune de ces substances, deuxièmement pour permettre, pendant un second intervalle de temps, leur mise en présence, et troisièmement pour permettre, au cours d'un troisième intervalle de temps, l'utilisation de ce mélange liquide évacué hors du dispositif de conditionnement, dans lequel l'ampoule est fermée à une de ses extrémités, ouverte à son autre extrémité et comporte au moins une première zone cylindrique (21) de section circulaire rétrécie pour définir un rétrécissement à l'intérieur de cette ampoule et au moins une deuxième zone cylindrique (23) de section circulaire plus large que la section de la première zone (21) dans lequel, pendant le premier intervalle de temps correspondant à la phase de stockage, ladite zone cylindrique de section circulaire rétrécie est obturée d'une part de façon étanche par ledit bouchon intermédiaire mobile (25) de manière à menager à l'intérieur de l'ampoule un premier compartiment (26) disposé entre l'extrémité fermée de cette ampoule et ledit bouchon intermédiaire mobile (25) et un second compartiment (28) disposé entre ledit bouchon intermédiaire mobile et l'extrémité ouverte de cette ampoule, et d'autre part par un piston-vanne (17) agencé pour fermer l'extrémité ouverte de l'ampoule; dans lequel, pendant ledit second intervalle de temps correspondant à la phase de mise en présence des deux substances, ledit bouchon intermédiaire mobile (25) est refoulé de la zone cylindrique de section circulaire rétrécie dans une zone cylindrique de section circulaire plus large pour permettre la communication entre le premier et le second compartiment et la mise en presence en milieu fermé des substances qu'ils contiennent, ladite zone cylindrique de section circulaire rétrécie restante obturée par le piston-vanne, et dans lequel, pendant ledit troisième intervalle de temps, ledit piston-vanne est pousse à l'intérieur de l'ampoule pour provoquer l'évacuation du mélange liquide hors de l'ampoule, caractérisé en ce que chaque section transversale du bouchon intermédiaire mobile (25) présente, à l'état détendu dans la zone cylindrique de section circulaire large (23), une forme différente de la forme circulaire, une surface inférieure à la surface de la section transversale de ladite zone de section large et au moins une dimension transversale égale au diamètre de ladite zone de section large (23), et en ce que le bouchon intermédiaire mobile (25) présente à l'état comprimé dans la zone cylindrique de section circulaire rétrécie une forme circulaire dont le diamètre est égal à celui de la section transversale de cette zone.

2. Dispositif selon la revendication 1, caractérisé en ce que l'ampoule (10) comporte une seule zone cylindrique de section circulaire rétrécie (21) ménagée du côté de son extrémité ouverte et une seule zone cylindrique de section circulaire large (23) ménagée du côté de son extrémité fermée, cette zone cylindrique de section circulaire rétrécie (21) comprenant essentiellement second compartiment (29) et cette zone cylindrique de section circulaire large comprenant essentiellement ledit premier compartiment (26).

3. Dispositif selon la revendication 1, caractérisé en ce que l'ampoule (10) comporte une zone cylindrique unique de section circulaire rétrécie (21) ménagée entre une première zone cylindrique de section circulaire large (20) et une seconde zone cylindrique de section circulaire large (23), ladite zone cylindrique de section circulaire rétrécie comprenant essentiellement ledit second compartiment et la seconde zone cylindrique de section circulaire large comprenant essentiellement ledit premier compartiment.

4. Dispositif selon la revendication 2 ou 3, caractérisé en ce que ladite zone cylindrique de section circulaire rétrécie (21) comporte trois secteurs (21a, 21b, 21c) dont le premier (21a) a un diamètre adapte pour assurer un blocage étanche du piston-vanne (17) pendant la phase de stockage, dont le deuxième (21b) définit ledit second compartiment, et dont le dernier (21c) a un diamètre adapté pour assurer un blocage étanché du bouchon intermédiaire mobile (25) pendant cette même phase de stockage.

5. Dispositif selon la revendication 1, caractérisé en ce que l'ampoule (10') comporte un première zone cylindrique de section circulaire large (20'), une première zone cylindrique de section circulaire rétrécie (21'a), une second zone cylindrique de section circulaire large (21'b), une seconde zone cylindrique de section circulaire rétrécie (21'c) et une troisième zone cylindrique de section circulaire large (23'), la première zone cylindrique de section circulaire rétrécie (21'a) ayant un diamètre adapte pour assurer un blocage étanche du piston-vanne (17) pendant la phase de stockage et la seconde zone cylindrique de section circulaire rétrécie (21'c) ayant un diamètre adapté pour assurer un blocage étanche du bouchon intermédiaire mobile (25) pendant cette même phase, les zones cylindriques de section, circulaire large (21'b) et (23') définissant respectivement ledit second et ledit premier compartiment.

6. Dispositif selon la revendication 1, caractérisé en ce que le bouchon intermédiaire mobile (25) présente une forme générale cylindrique et que sa surface latérale a une section au moins approximativement polygonale à l'état détendu.

7. Dispositif selon la revendication 1, caractérisé en ce que le bouchon intermédiaire mobile (25) présente une forme générale cylindrique et que sa surface latérale a une section approximativement ovale à l'état détendu.

8. Dispositif selon la revendication 1, caractérisé en ce que l'organe d'obturation (19) présente une forme générale cylindrique et en ce que sa surface latérale a une section au moins approximativement polygonale à l'état détendu.

9. Dispositif selon la revendication 1, caractérisé en ce que l'organe d'obturation (19) présente une forme générale cylindrique et en ce que sa surface latérale a une section au moins approximativement ovale à l'état détendu.

10. Dispositif selon l'une quelconque des revendications 6 ou 7, caractérisé en ce que le bouchon intermédiaire mobile (25) présente une entaille ménagée sur une partie de sa hauteur, cette entaille débouchant dans la surface disposée en regard du fond de l'ampoule (10).

## Patentansprüche

1. Vorrichtung zum Aufbewahren von flüssigen oder von flüssigen und festen Substanzen, bestehend aus einer Ampulle (10) von zylindrischer länglicher Gesamtform mit kreisförmigem Querschnitt, mit zwei in gegenseitiger Verlängerung angeordneten und durch einen beweglichen, aus einem Elastomer bestehenden Zwischenstopfen (25) getrennten Abteilen (26, 28), wovon jedes mindestens eine unterschiedliche Substanz enthält und die zwei Substanzen erhalten bleiben, bis man sie miteinander in Verbindung bringt, um eine flüssig Mischung zur medizinischen oder paramedikalen Anwendung zu bilden, wobei diese Vorrichtung geschaffen ist, um erstens während einer ersten Zeitspanne für jede dieser Substanzen Lagerungsbedingungen zu gewährleisten, zweitens um während einer zweiten Zeitspanne ihr Zusammenbringen zu ermöglichen und drittens, um im Verlauf einer dritten Zeitspanne die Anwendung dieser aus der Vorrichtung zum Aufbewahren entnommenen Mischung zu ermöglichen, eine Vorrichtung, bei welcher die Ampulle an einem ihrer Enden geschlossen, an ihrem anderen Enden offen ist, und die mindestens einen ersten zylindrischen Abschnitt (21) von eingeschnürtem, kreisförmigem Querschnitt zur Abgrenzung einer Verengung im Innern dieser Ampulle aufweist, und mindestens einen zweiten zylindrischen Abschnitt (23) von größerem, kreisförmigem Querschnitt als der Querschnitt in dem ersten Abschnitt (21), wobei während der ersten, der Lagerungsphase entsprechenden Zeistspanne dieser zylindrische Abschnitt mit eingeschnürtem Querschnitt einerseits durch diesen beweglichen Zwischenstopfen (25) dicht abgeschlossen ist, so daß sich im Innern der Ampulle ein erstes zwischen dem geschlossenen Ende der Ampulle unter dem beweglichen Zwischenstopfen (25) liegendes Abteil (26) und ein zwischen dem beweglichen Zwischenstopfen und dem offenen Ende der Ampulle liegendes zweites Abteil (28) bildet, und der andererseits dicht abgeschlossen ist durch ein Kolbenventil (17), welches dazu dient, das offene Ende der Ampulle zu verschließen; wobei man während der zweiten, dem Zusammenbringen der zwei Substanzen entsprechenden Zeitspanne den beweglichen Zwischenstopfen (25) aus dem zylindrischen Abschnitt mit eingeschnürtem kreisförmigem Querschnitt in einen zylindrischen Abschnitt mit kreisförmigem größerem Querschnitt schiebt, um die Verbindung zwischen dem ersten und dem zweiten Abteil herzustellen, und um in einem abgeschlossenen Milieu das Zusammenbringen der in ihren befindlichen Substanzen zu ermöglichen, während der zylindrische Abschnitt mit eingeschnürtem Querschnitt durch das Kolbenventil verschlossen bleibt, und wobei man während der dritten Zeitspanne das Kolbenventil in die Ampulle hineindrückt, um das Austreten der flüssigen Mischung aus der Ampulle zu veranlassen, dadurch gekennzeichnet, daß jeder Querschnitt des beweglichen Zwischenstopfens (25) im entspannten Zustand in dem zylindrischen Abschnitt mit großem kreisförmigem Querschnitt (23) eine von der kreisförmigen Form abweichende Form aufweist, des weiteren eine Fläche, die kleiner ist als die Fläche des Querschnitts des Abschnittes mit großem Querschnitt, und mindestens eine Querdimension, die gleich dem Durchmesser des Abschnittes mit großem Querschnitt (33) ist, und daß der bewegliche Zwiscenstopfen (25) im zusammengedrückten Zustand in dem zylindrischen Abschnitt mit eingeschnürtem Querschnitt eine kreisförmige Form aufweist, deren Durchmesser gleich dem des Querschnitts dieses Abschnitts ist.

2. Vorrichtung nach Anspruch 1, dadurch gekennzeichnet, daß die Ampulle (10) einen einzigen zylindrischen Abschnitt mit eingeschnürtem kreisförmigem Querschnitt (21), vorgesehen an ihrem offenen Ende, aufweist und einen einzigen bodenseitig vorgesehenen zylindrischen Abschnitt mit großem kreisförmigem Querschnitt (23), wobei der zylindrische Abschnitt mit eingeschnürtem kreisförmigem Querschnitt (21) hauptsächlich das zweite Abteil (29) und der zylindrische Abschnitt mit großem kreisförmigem Querschnitt hauptsächlich das erste Abteil (26) umfaßt.

3. Vorrichtung nach Anspruch 1, dadurch gekennzeichnet, daß die Ampulle (10) einen einzigen zylindrischen Abschnitt mit eingeschnürtem kreisförmigem Querschnitt (21) aufweist, vorgesehen zwischen einem ersten zylindrischen Abschnitt mit großen kreisförmigem Querschnitt (20) und einem zweiten zylindrischen Abschnitt mit großem kreisförmigem Querschnitt (23), wobei der zylindrische Abschnitt mit eingeschnürtem kreisförmigem Querschnitt hauptsächlich das zweite Abteil und der zweite zylindrische Abschnitt mit großem kreisförmigem Querschnitt hauptsächlich das erste Abteil umfaßt.

4. Vorrichtung nach Anspruch 1 oder 2, dadurch gekennzeichnet, daß der zylindrische Abschnitt mit eingeschnürtem kreisförmigem Querschnitt (21) drei Abschnitte (21a, 21b, 21c) aufweist, wovon der erste (21a) einen passenden Durchmesser hat, um während des Lagerungszeitraumes einen dichten Sitz des Kolbenventils (17) zu gewährleisten, der zweite (21b) das zweite Abteil bildet, und der letzte (21c) einen passenden Durchmesser hat, um ebenfalls während des Lagerungszeitraumes eine dichten Sitz des beweglichen Zwischenstopfens (25) zu gewährleisten.

5. Vorrichtung nach Anspruch 1, dadurch gekennzeichnet, daß die Ampulle (10') einen

9

ersten zylindrischen Abschnitt mit großem kreisförmigem Querschnitt (20') aufweist, einen ersten zylindrischen Abschnitt mit eingeschnürtem kreisförmigem Querschnitt (21'a), einen zweiten zylindrischen Abschnitt mit großem kreisförmigem Querschnitt (21'b), einen zweiten zylindrischen Abschnitt mit eingeschnürtem kreisförmigem Querschnitt (21'c) und einen dritten zylindrischen Abschnitt mit großem kreisförmigem Querschnitt (23'), wobei der erste zylindrische Abschnitt mit eingeschnürtem kreisförmigem Querschnitt (21'a) einen passenden Durchmesser hat, um während des Lagerungszeitraumes einen dichten Sitz des Kolbenventils (17) zu gewährleisten und der zweite zylindrische Abschnitt mit eingeschnürtem Querschnitt (21'c) einen passenden Durchmesser, um während desselben Zeitraumes einen dichten Sitz des beweglichen Zwischenstopfens (25) zu gewährleisten, wobei die zylindrischen Abschnitte mit großem kreisförmigem Querschnitt (21'b) und (23') jeweils das zweite und das erste Abteil bilden.

6. Vorrichtung nach Anspruch 1, dadurch gekennzeichnet, daß der bewegliche Zwischenstopfen (25) eine zylindrische Gesamtform aufweist und daß seine Mantelfläche im entspannten Zustand mindestens annähernd polygonalen Querschnitt hat.

7. Vorrichtung nach Anspruch 1, dadurch gekennzeichnet, daß der bewegliche Zwischenstopfen (25) eine zylindrische Gesamtform aufweist und daß seine Mantelfläche im entspannten Zustand einen annähernd ovalen Querschnitt hat.

8. Vorrichtung nach Anspruch 1, dadurch gekennzeichnet, daß das Verschlußorgan (19) eine zylindrische Gesamtform aufweist und daß seine Mantelfläche im entspannten Zustand einen mindestens annähernd polygonalen Querschnitt hat.

9. Vorrichtung nach Anspruch 1, dadurch gekennzeichnet, daß das Verschlußorgan (19) eine zylindrische Gesamtform aufweist und daß seine Mantelfläche im entspannten Zustand einen mindestens annähernd ovalen Querschnitt hat.

10. Vorrichtung nach einem beliebigen Anspruch 6 o. 7, dadurch gekennzeichnet, daß der bewegliche Zwischenstopfen (25) eine auf einem Teil seiner Höhe angebrachte Nut aufweist, wobei diese Nut an der Stirnfläche austritt, die dem Boden der Ampulle gegenüberliegt.

## Claims

1. Conditioning device for liquid substances or liquids and solids, comprising a generally cylindrical, elongate ampoule (10) of circular section, with two compartments (26, 28) each disposed in the extension of the other separated by an intermediate movable, elastomeric stopper (25), each compartment containing at least one different substance, the two substances being stored together to later form a liquid mixture for medical or paramedical purposes, said device being designed, initially, to provide storage during a first stage for each of the substances, secondly to ensure, during a second stage, their admixture, and thirdly to provide for use of the liquid mixture evacuated from the conditioning device during a third stage, in which the ampoule is closed at one end, open at the other end and comprises at least one cylindrical zone (21) of narrowed circular section, tapering to form a smaller area inside the ampoule and at least a second cylindrical zone (23) with a circular section wider than the section of the first zone (21) wherein, during the first time interval corresponding to the storage phase, the said cylindrical zone of narrowed circular section is tightly sealed at one end by said movable intermediary plug (25) so as to form a first compartment (26) inside the ampoule disposed between the closed end of said ampoule and said movable intermediary plus (25) and a second compartment (28) disposed between said movable intermediary plug and the open end of said ampoule, and at the other end by a piston-valve (17) designed to close the open end of the ampoule wherein, during the second stage of mixing the two substances, said movable intermediary plug (25) is forced back from the narrower circular cylindrical zone to the wider circular cylindrical zone to allow communication between the first and second compartments and mixing of the substances contained therein in a closed area, said narrowed circular cylindrical zone remaining stopped by the piston-valve and wherein, during the time third interval, said piston-valve is forced inside the ampoule to evacuate the liquid mixture from the ampoule, characterized in that each transverse section of the movable intermediary plug (25) has, in its distended state in the cylindrical zone of wide circular section (23), a shape other than circular, a smaller surface than the transverse surface of the said wide section zone and at least one transverse dimension equal to the diameter of the said wide section zone (23), and in that the movable intermediary plug (25), in its compressed state in the cylindrical zone of narrowed circular section, is circular with a diameter equal to that of the transverse section of said zone.

2. Device according to claim 1, characterized in that the ampoule (10) comprises a single cylindrical zone of narrowed circular section (21) disposed near its open extremity and a single cylindrical zone of wide circular section (23) disposed near its closed end, said cylindrical zone of narrowed circular section (21) essentially comprising the said second compartment (29) and said cylindrical zone of wide circular section essentially comprising the said first compartment (26).

3. Device according to claim 1, characterized in that the ampoule (10) comprises a single cylindrical zone of narrowed circular section (21) disposed between a first cylindrical zone of wide circular section (20) and a second cylindrical zone of wide circular section (23), said circular zone of narrowed circular section essentially comprising the said second compartment and the second cylindrical zone of wide circular section essentially comprising the said first compartment.

4. Device according to claim 2 or 3, characterized in that the said cylindrical zone of narrowed circular section (21) comprises three sections (21a, 21b, 21c), the first of which (21a) has a diameter adapted to ensure tight sealing of the piston-valve (17) during the storage phase, the second of which (21b) defines the said second compartment, and the last of which (21c) has a diameter adapted to ensure complete sealing of the movable intermediary plug (25) during said same storage phase.

5. Device according to claim 1, characterized in that the ampoule (10') comprises a first cylindrical zone of wide circular section 20'), a first cylindrical zone of narrowed circular section (21'a), a second cylindrical zone of wide circular section (21'b), a second cylindcical zone of narrowed circular section (21'c) and a third cylindrical zone of wide circular section (23'), the first cylindrical zone of narrowed circular section (21'a) having a diameter adapted to ensure tight sealing of the piston-valve (17) during the storage phase and the second cylindrical zone of narrowed circular section (21'c) having a diameter adapted to ensure tight sealing of the movable intermediary plug (25) during the same phase, the cylindrical zones of wide circular section (21'b) and (23')

defining the said second and the said first comparment, respectively.

6. Device according to claim 1, characterized in that the movable intermediary plug (25) is of generally cylindrical shape and that its lateral surface has an at least approximately polygonal cross section in its distended state.

7. Device according to claim 1, characterized in that the movable intermediary plug (25) is of generally cylindrical shape and that its lateral surface has an approximately oval cross section in its distended state.

8. Device according to claim 1, characterized in that the stopper means (19) has a generally cylindrical shape and in that its lateral surface has an approximately polygonal cross section in its distended state.

9. Device according to claim 1, characterized in that the stopper means (19) has a generally cylindrical shape and in that its lateral surface has an approximately oval cross section in its distended state.

10. Device according to any one of claims 7 or 8, characterized in that the movable intermediary plug (25) has an opening disposed in part of its upper portion, said opening leading into the surface disposed across from the base of the ampoule (10).

Fig. I

Fig. II

Fig. 2

Fig. 4

Fig. 5

Fig. 6

2

Fig. 3b

Fig. 3c

Fig. 3a

Fig. 8

Fig. 7

Fig. 10